# EUROPÄISCHE PATENTANMELDUNG

(11) **EP 0 719 771 A1**
(43) Veröffentlichungstag der Anmeldung: **03.07.1996**
(21) Anmeldenummer: 95119065.1
(22) Anmeldetag: 04.12.1995
(51) Int. Cl.: C07D 333/12, C07D 333/08, C07D 307/36

(54) **Verfahren zur Herstellung von Methylenverbindungen und die Verbindung 2-(2',4'-Dichlor-5'-fluorbenzyl)-thiophen**

(30) Priorität: 16.12.1994 DE 4444862
(71) Anmelder: BAYER AG, 51368 Leverkusen (DE)
(72) Erfinder: Fiege, Helmut, Dr., D-51373 Leverkusen (DE); Hagedorn, Ferdinand, Dr., D-51381 Leverkusen (DE)

(57) **Zusammenfassung**

Methylenverbindungen werden vorteilhafterweise hergestellt, indem man einen Aluminiumhalogenid-Komplex der Formel 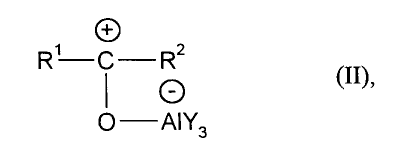
in der
- R¹: für einen gegebenenfalls durch 1 bis 4 C₁-C₄-Alkylreste, 1 bis 4 Fluor-, Chlor- und/oder Bromatome, eine C₁-C₈-Alkoxygruppe und/oder eine Acetoxygruppe substituierten C₆-C₁₀-Arylrest oder einen gegebenenfalls durch einen C₁-C₄-Alkylrest und/oder ein Fluor-, Chlor- oder Bromatom substituierten 5 bis 10 C-Atome und ein O- oder S-Atom enthaltenden Heteroarylrest und
- R²: für einen gegebenenfalls durch 1 bis 5 Fluor-, Chlor- und/oder Bromatome substituierten C₁-C₁₂-Alkylrest, unabhängig von R¹ für einen Rest wie bei R¹ definiert und im Falle R¹ = ein mit 1 bis 4 Fluor-, Chlor- und/oder Bromatomen substituierter C₆-C₁₀-Arylrest, auch für einen Furyl- oder Thienylrest stehen,
- Y: für Fluor, Chlor oder Brom steht,
mit einem Aminboran der Formel 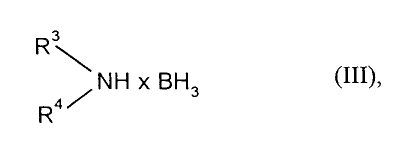
in der
- R³: für C₁-C₄-Alkyl und
- R⁴: für Wasserstoff oder C₁-C₄-Alkyl stehen
und/oder
mit Natrium- und/oder mit Kaliumborhydrid reduziert.

Erstmals zugänglich ist so 2-(2',4'-Dichlor-5-fluorbenzyl)-thiophen.

## Beschreibung

Die vorliegende Erfindung betrifft ein besonders vorteilhaftes Verfahren zur Herstellung von Methylenverbindungen. Methylenverbindungen sind wichtige Zwischenprodukte zur Herstellung der verschiedensten Wirkstoffe. Die vorliegende Erfindung betrifft auch das mit diesem Verfahren erstmals zugänglich gewordene 2-(2',4'-Dichlor-5-fluorbenzyl)-thiophen. Beispielsweise stellen 2-(4'-Fluorbenzyl)-thiophen und 2-(4'-Fluorbenzyl)-furan wichtige Bausteine zur Herstellung von Leukotrien-Biosynthese-Inhibitor-Wirkstoffen dar (s. US-A 5 288 571).

Es ist bekannt, daß man Methylenverbindungen durch die Reduktion von Ketonen nach Wolff-Kishner und Huang Minlon herstellen kann (s. J. March, Advanced Organic Chemistry, McGraw-Hill, S. 1119-1121 (1977)). Diese Methoden erfordern die Handhabung von Hydrazin, was man aus ökologischen Gründen zu vermeiden sucht. Außerdem versagen solche Reduktionsverfahren bei Ketonen, deren Hydrazone nur schwer herstellbar sind und in solchen Fällen, bei denen auch Substituenten der Ketone mit Hydrazin reagieren können.

Ferner ist die Reduktion von Ketonen mit Natriumborhydrid und tert.-Butylamin-boran in Gegenwart von wasserfreiem Aluminiumchlorid bekannt (s. Synthesis, S. 736-738 (1987) und J. Org. Chem. 54, 491-494 (1989)). Die bei diesen Verfahren erforderlichen Mengen an Aluminiumchlorid und vor allem an Natriumborhydrid oder tert.-Butylamin-boran liegen mit 3 bis 5 mol/mol Keton außerordentlich hoch, so daß dieses Verfahren wegen der benötigten Mengen an Hilfsstoffen nachteilig ist.

Auch die Reduktion von Ketonen mit Natriumborhydrid in Trifluoressigsäure als Reaktionsmedium ist beschrieben (s. Synthesis, S. 763-765 (1978)). Hierbei sind die technische Handhabung von Trifluoressigsäure aufgrund ihrer Korrosivität, ihrer Wasserlöslichkeit und der Azeotropbildung mit Wasser sowie ihr hoher Preis problematisch.

2-(4'-Fluorbenzyl)-thiophen ist auf einem anderen Syntheseweg hergestellt worden durch Einwirkung von 2-Lithiumthiophen auf 4-Fluorbenzylbromid in Gegenwart von Tetrakis-(triphenylphosphin)-palladium(0) bei -78°C in Diethylether/Tetrahydrofuran. Die Ausbeute ist mit 81 % zwar gut, die Herstellungsmethode jedoch äußerst aufwendig und für eine technische Anwendung wenig geeignet (s. US-P 5 288 751, Beispiel 12A).

Ketone kann man durch Acylierung von Säurehalogeniden oder Säureanhydriden mit Aromaten oder heteroaromatischen Verbindungen mit Aluminiumtrihalogeniden nach Friedel-Crafts herstellen. Hierbei entsteht im Verlauf der Reaktion unter Chlorwasserstoffabspaltung in vielen Fällen ein in dem jeweils verwendeten Reaktionsmedium löslicher Aluminiumhalogenid-Komplex. Dieser Aluminiumhalogenid-Komplex wird zur Gewinnung des Ketons dann üblicherweise durch Eintragen in Eiswasser hydrolysiert. So erhaltenes Keton läßt sich mit Borwasserstoffverbindungen allein nur bis zur Carbinolstufe, nicht jedoch bis zur Methylenstufe reduzieren (s. Houben-Weyl, Methoden der organischen Chemie, Band 4/1d, S. 267 (1981)).

Es wurde nun ein Verfahren zur Herstellung von Methylenverbindungen der Formel

R¹-CH₂-R² (I),

in der
- R¹: für einen gegebenenfalls durch 1 bis 4 C₁-C₄-Alkylreste, 1 bis 4 Fluor-, Chlor- und/oder Bromatome, eine C₁-C₈-Alkoxygruppe und/oder eine Acetoxygruppe substituierten C₆-C₁₀-Arylrest oder einen gegebenenfalls durch einen C₁-C₄-Alkylrest und/oder ein Fluor-, Chlor- oder Bromatom substituierten 5 bis 10 C-Atome und ein O- oder S-Atom enthaltenden Heteroarylrest und
- R²: für einen gegebenenfalls durch 1 bis 5 Fluor-, Chlor- und/oder Bromatome substituierten C₁-C₁₂-Alkylrest, unabhängig von R¹ für einen Rest wie bei R¹ definiert und im Falle R¹ = ein mit 1 bis 4 Fluor-, Chlor- und/oder Bromatomen substituierter C₆-C₁₀-Arylrest, auch für einen Furyl- oder Thienylrest stehen,
gefunden, das dadurch gekennzeichnet ist, daß man einen Aluminiumhalogenid-Komplex der Formel 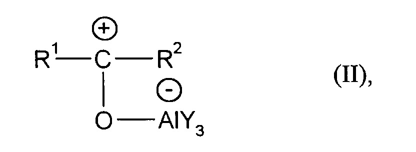
in der

- R¹ und R²: die oben angegebene Bedeutung haben und
- Y: für Fluor, Chlor oder Brom steht,
mit einem Aminboran der Formel 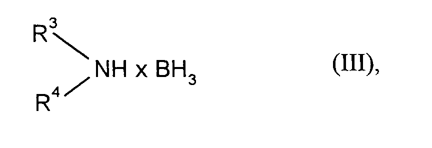
in der
- R³: für C₁-C₄-Alkyl und
- R⁴: für Wasserstoff oder C₁-C₄-Alkyl stehen
und/oder
mit Natrium- und/oder mit Kaliumborhydrid reduziert.

Aluminiumhalogenid-Komplexe der Formel (II) sind prinzipiell auf zwei Wegen zugänglich, nämlich
a) durch Friedel-Crafts-Acylierung eines Aromaten oder Heteroaromaten der Formel

   R¹-H (IV),

   in der
   - R¹: die oben angegebene Bedeutung hat,
   mit Acylverbindungen der Formel

   R²-COX (V),

   in der
   - R²: die oben angegebene Bedeutung hat und
   - X: für Fluor, Chlor oder Brom steht,
oder
b) durch Friedel-Crafts-Acylierung einer Verbindung der Formel

   R²-H (VI),

   in der
   - R²: die oben angegebene Bedeutung hat,
   mit einer Acylverbindung der Formel

   R¹-COX (VII),

   in der
   - R¹ und X: die oben angegebenen Bedeutungen haben.

Nicht in allen Fällen führen beide Wege zum Erfolg, jedoch immer mindestens einer. Wenn R² für einen Alkyl- oder Arylrest steht, ist der Weg a) bevorzugt, wenn R² für einen Furyl- oder Thienylrest steht der Weg b).

Diese Verfahrensweise der Herstellung des Aluminiumhalogenid-Komplexes der Formel (II) und dessen erfindungsgemäße Reduktion bietet den Vorteil, bisher notwendige zusätzliche Reaktionsschritte, z.B. die Aufarbeitung, Isolierung, Reinigung und Trocknung des jeweiligen Ketons; den erneuten Einsatz von Aluminiumhalogenid bei der Reduktion; mehrstufige Arbeitsweise; Handhabung großer Mengen an Hilfsstoffen; Handhabung korrosiver und teurer Chemikalien zu vermeiden. Eine Isolierung des Aluminiumhalogenid-Komplexes der Formel (II) ist nicht erforderlich. Er kann in Form des bei seiner Herstellung anfallenden Reaktionsgemisches weiterverarbeitet werden.

Das erfindungsgemäße Verfahren ist einstufig, geht von gut zugänglichen Ausgangsverbindungen aus, erfordert nur relativ geringe Mengen an Hilfsstoffen und benötigt keine korrosiven und/oder teuren Chemikalien.

In den Formeln (I), (II), (IV) und (VII) steht R¹ vorzugsweise für einen unsubstituierten oder mit 1 bis 4 Fluor-, Chlor- und/oder Bromatomen substituierten Phenylrest oder einen 5 C-Atome und ein S- oder O-Atom enthaltenden Heteroarylrest.

In den Formeln (I), (II), (V) und (VI) steht R² vorzugsweise für einen Phenylrest, der gegebenenfalls 1 bis 3 Substituenten aus der Gruppe Fluor und Chlor enthält oder wenn R¹ für einen mit 1 bis 4 Fluor-, Chlor- und/oder Bromatomen substituierten C₆-C₁₀-Arylrest steht, auch für einen Furyl- oder Thienylrest.

Vorzugsweise steht in den Formeln (V) und (VII) X für Chlor oder Brom und unabhängig davon Y in Formel (III) ebenfalls für Chlor oder Brom.

In Formel (III) steht R³ vorzugsweise für Methyl, Ethyl oder tert.-Butyl und R⁴ vorzugsweise für Wasserstoff, Methyl oder Ethyl.

Besonders bevorzugte Ausgangsprodukte der Formeln (IV) bzw. (VI) sind Thiophen, Benzothiophen, Benzofuran, Fluorbenzol und 2,4-Dichlorfluorbenzol..

Besonders bevorzugte Ausgangsprodukte der Formeln (V) bzw. (VII) sind 4-Fluorbenzoylchlorid, 4-Chlorbenzoylchlorid, 2,4-Dichlor-5-fluorbenzoylchlorid, 2-Chlor-4,5-difluorbenzoylchlorid, Thenoylchlorid und Furoylchlorid.

Besonders bevorzugte Aminborane der Formel (III) sind Dimethylaminboran und tert.-Butylaminboran. Der Einsatz von Natrium- und Kaliumborhydrid ist ebenfalls bevorzugt.

Besonders bevorzugt werden mit dem erfindungsgemäßen Verfahren folgende Methylenverbindungen der Formel (I) hergestellt: 2-(4'-Fluorbenzyl)-thiophen, 2-(4'-Chlorbenzyl)-thiophen, 2-(4-Fluorbenzyl)-furan, 2-(2',4'-Dichlor-5-fluorbenzyl)-thiophen, 2-(2'-Chlor-4,5-difluorbenzyl)-thiophen und 2-(2,4-Dichlor-5-fluorbenzyl)-furan.

Das erfindungsgemäße Verfahren kann man beispielsweise nach folgender Methode durchführen:
Eine Acylverbindung der Formel (V) bzw. (VII) wird mit einer Verbindung der Formel (IV) bzw. (VI) unter Zusatz eines Aluminiumhalogenids in einem inerten Verdünnungsmittel unter Abspaltung von Halogenwasserstoff umgesetzt. Nach Entfernung des Halogenwasserstoffs, z.B. durch Anlegen von Vakuum, setzt man das erhaltene Reaktionsgemisch, das einen Aluminiumhalogenid-Komplex der Formel (II) enthält, mit einem Aminboran der Formel (III) oder Natrium- oder Kaliumborhydrid um, hydrolysiert das Gemisch zur Eliminierung des Aluminiumhalogenids mit Wasser, trennt die Wasserphase ab und destilliert die organische Phase.

Als inerte Verdünnungsmittel kommen bei Friedel-Craft-Acylierungen übliche Lösungsmittel in Frage, die zusätzlich gegenüber Borwasserstoffverbindungen inert sein müssen. Bevorzugt sind Halogenbenzole und Ether, insbesondere Chlorbenzol, o-Dichlorbenzol und Gemische aus Chlorbenzol und Diglyme.

Bis zu der Stufe der den Aluminiumhalogenid-Komplex der Formel (II) enthaltende Reaktionsgemische kann man so verfahren, wie es für die Herstellung von Ketonen aus Aromaten und Acylverbindungen nach Friedel-Crafts bekannt ist (s. z.B. Houben-Weyl, Methoden der organischen Chemie, Band VII/2a, S. 15 ff. (1973)).

Die erfindungsgemäße Reduktion von Aluminiumhalogenid-Komplexen der Formel (II) mit einer der angegebenen Borwasserstoffverbindungen kann man z.B. bei 0 bis 100°C, vorzugsweise bei 50 bis 90°C durchführen. Es ist vorteilhaft, bei dieser Reduktion ein zusätzliches Lösungsmittel einzusetzen, z.B. einen Ether, insbesondere wenn die Borwasserstoffverbindung im sonst vorliegenden Reaktionsmedium schlecht löslich ist. Bevorzugte zusätzliche Lösungsmittel sind Tetrahydrofuran, Diethylenglykoldimethylether (Diglyme) und Ethylenglykoldimethylether.

Vorzugsweise setzt man die Lösung oder Suspension, die den Aluminiumhalogenid-Komplex der Formel (II) enthält, einer Lösung oder Suspension, die die Borwasserstoffverbindung in z.B. Diglyme enthält, zu..

Die Reaktionstemperatur kann bei den Reaktionsschritten der Acylierung und der Reduktion durchaus unterschiedlich sein. So kann beispielsweise die Reaktion einer Acylverbindung der Formel (V) bzw. (VII) mit einer Verbindung der Formel (IV) bzw. (VI) anfangs im niedrigen Temperaturbereich, z.B. bei -10 bis +10°C beginnen und im Verlauf der Umsetzung auf 50 bis 110°C angehoben werden. Dagegen kann man die Reduktion im Temperaturbereich von 0 bis 100°C, bevorzugt 50 bis 90°C durchführen.

Die pro mol Aluminiumhalogenid-Komplexe der Formel (II) einzusetzende Menge Borwasserstoffverbindung kann beispielsweise 0,5 bis 1 mol, bevorzugt 0,7 bis 0,8 mol betragen. Ein größerer Überschuß des Reduktionsmittels verbessert das Ergebnis im allgemeinen nicht, sondern bringt die Gefahr der Perhydrierung von aromatischen bzw. heterocyclischen Resten mit sich.

Mit dem erfindungsgemäßen Verfahren ist es erstmals möglich, 2-(2',4'-Dichlor-5-fluorbenzyl)-thiophen der Formel 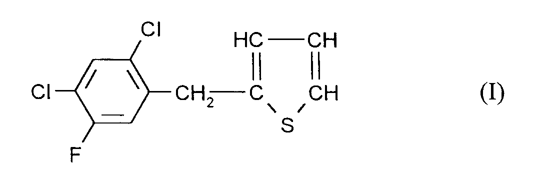
herzustellen (siehe Beispiel 4). Die vorliegende Erfindung betrifft deshalb auch die Verbindung der Formel (I). Man kann aus der Verbindung der Formel (I) Lipoxygenase-Inhibitoren und Inhibitoren für Leukotrien-Biosynthesen herstellen, so wie es für andere 2-Benzylthiophene bekannt ist (siehe WO 90/12008 und US-A 5 288 751).

### Beispiele

### Beispiel 1 2-(4'-Fluorbenzyl)-thiophen

33,8 g Aluminiumtrichlorid wurden in 217 g Chlorbenzol suspendiert und dazu eine Lösung von 40,3 g 4-Fluorbenzoylchlorid in 37,4 g Chlorbenzol innerhalb von 15 Minuten bei 0°C zugetropft. Das Gemisch wurde 1 Stunde nachgerührt und dann bei 0°C 21,8 g Thiophen innerhalb von 20 Minuten zugetropft. Der entstehende Chlorwasserstoff wurde abgeleitet und in Natronlauge absorbiert. Anschließend wurde das Gemisch auf 80°C erhitzt und der restliche Chlorwasserstoff entfernt. Die erhaltene Lösung wurde zu einer Suspension von 7,2 g Natriumborhydrid in 47,2 g Diglyme innerhalb von 45 Minuten bei 70°C unter Rühren zugetropft. Das Reaktionsgemisch wurde 90 Minuten nachgerührt, dann auf Raumtemperatur abgekühlt und auf ein Gemisch aus 250 g Eis, 44,6 g konz. wäßrige Salzsäure und 500 g Wasser ausgetragen. Die dabei sich bildende organische Phase wurde abgetrennt, die Wasserphase mit Chlorbenzol ausgeschüttelt und die vereinigten organischen Phasen destilliert. Es wurden 35,0 g Produkt mit einem Siedepunkt von 83°C/1,0 mbar und einer Reinheit von 99,7 % (GC) erhalten. Das entspricht 73,9 % der Theorie. Das Produkt enthielt weniger als 0,1 Gew.-% entfluorierte Verbindungen (GC).

### Beispiel 2 2-Benzylthiophen

In 130 ml Chlorbenzol wurden 27,2 g 98,5 gew.-%iges Aluminiumtrichlorid (sublimiert) suspendiert und auf 0°C abgekühlt. Innerhalb von 30 Minuten wurden 28,7 g Benzoylchlorid unter Rühren und Kühlen zugetropft und dann 1 Stunde nachgerührt. Ebenfalls bei 0°C wurde eine Lösung von 17,3 g Thiophen in 27 ml Chlorbenzol innerhalb von 30 Minuten zugetropft und 1 Stunde nachgerührt. Unter Erwärmen des Gemisches und Rühren wurde restlicher Chlorwasserstoff ausgetrieben, am Ende im Vakuum. Die verbleibende Lösung wurde innerhalb von 40 Minuten in eine Lösung von 12,7 g Dimethylaminboran in 70 ml Chlorbenzol unter Rühren bei 70°C eingetragen. Das Gemisch wurde 1 Stunde nachgerührt, abgekühlt und auf eine Mischung von 200 g Eis und 50 g konz. wäßriger Salzsäure unter Rühren ausgetragen. Danach wurde die Wasserphase abgetrennt und mit Chlorbenzol extrahiert. Die zurückbleibende organische Phase wurde mit Wasser ausgeschüttelt. Danach wurde die organische Phase und das Extrakt aus der Wasserphase vereinigt, das Lösungsmittel daraus abdestilliert und durch Destillation 38,7 g rohes 2-Benzylthiophen in 79,7 %iger Reinheit erhalten. Das entspricht 88,5 % der Theorie. Durch Redestillation wurde 99,7 % reines 2-Benzylthiophen mit einem Siedepunkt von 91 bis 92°C/1,4 mbar erhalten.

### Beispiel 3 2-(4'-Fluorbenzyl)-thiophen

33,8 g Aluminiumchlorid wurden in 200 g Fluorbenzol suspendiert, dazu wurde eine Lösung von 37,8 g Thiophen-2-carbonsäurechlorid in 50 ml Fluorbenzol innerhalb von 30 Minuten bei 0°C zugetropft. Das Gemisch wurde 2 Stunden bei 0°C gerührt, anschließend langsam erwärmt und 2 Stunden bei 85°C am Rückfluß erhitzt, bis die Chlorwasserstoffentwicklung beendet war. Nach Kühlen der Lösung auf 70°C wurde sie bei dieser Temperatur innerhalb von 30 Minuten in eine gerührte Suspension von 7,2 g Natriumborhydrid in 50 ml Diglyme zugetropft. Das Gemisch wurde 90 Minuten bei 70°C nachgerührt und nach dem Abkühlen auf eine Mischung aus 250 g Eis, 200 ml Wasser und 62,5 g. konz. wäßr. Salzsäure ausgetragen. Nach Zugabe von 100 ml Fluorbenzol wurde die organische Phase abgetrennt, mit 100 ml Wasser ausgeschüttelt und eingeengt. Ausbeute: 58,3 g Produkt mit einem Gehalt von 61,8 Gew.-% 2-(4'-Fluorbenzyl)-thiophen (GC), entsprechend einer Ausbeute von 75,1 % d. Th. Aus der wäßrigen Mutterlauge wurde durch Extraktion mit Methylenchlorid und Einengen eine organische Phase gewonnen, die noch weitere 33,7 Gew.-% des gewünschten Produkts enthielt (GC), entsprechend einer zusätzlichen Ausbeute von 4,6 % d. Th. Durch Destillation der organischen Phasen wurde ein 99,2 %iges 2-(4'-Fluorbenzyl)-thiophen gewonnen.

### Beispiel 4 (2-(2',4'-Dichlor-5-fluorbenzyl)-thiophen

33,8 g Aluminiumchlorid wurde in 162,5 ml Chlorbenzol suspendiert, dazu wurde eine Lösung von 58,9 g 2,4-Dichlor-5-fluorbenzoylchlorid in 33,8 ml Chlorbenzol innerhalb 30 Minuten bei 0°C getropft. Das Gemisch wurde 90 Minuten bei 0°C nachgerührt, danach wurde bei 0°C eine Lösung von 21,8 g Thiophen in 33,8 ml Chlorbenzol innerhalb 30 Minuten zugetropft. Das Gemisch wurde weitere 2 Stunden bei 0°C nachgerührt und am Ende 15 Minuten zum Rückfluß erhitzt, bis die Chlorwasserstoffentwicklung beendet war. Die erhaltene Lösung wurde zu einer auf 70°C erwärmten Suspension von 7,2 g Natriumborhydrid in 50 ml Diglyme innerhalb 30 Minuten zugetropft, das Reaktionsgemisch anschließend 90 Minuten bei 70°C nachgerührt, danach auf ein Gemisch aus 250 g Eis, 200 ml Wasser und 62,5 g konz. wäßr. Salzsäure ausgetragen. Nach Zugabe von 100 ml Chlorbenzol und Rühren wurden die Phasen getrennt. Die organische Phase wurde mit 100 ml Wasser ausgeschüttelt, anschließend eingeengt. Ausbeute: 78,5 g Rohsubstanz mit einem Gehalt von 76,7 Gew.-% (GC) an 2-(2',4'-Dichlor-5-fluorbenzyl)-thiophen entsprechend 92,3 % d. Th. Durch Destillation wurde ein 97,2 gew.-%iges (GC) Produkt, Sdp. 140 bis 145°C/8 mbar, Fp. 60 bis 61°C, erhalten.

### Beispiel 5 2-(4'-Methylbenzyl)-thiophen

Zu einer Suspension von 33,8 g Aluminiumchlorid in 162,5 ml Chlorbenzol wurden bei 0°C innerhalb 30 Minuten unter Rühren 39 g 4-Methylbenzoylchlorid zugetropft. Das Gemisch wurde 90 Minuten bei 0°C nachgerührt, anschließend wurde bei 0°C innerhalb 30 Minuten eine Lösung von 21,8 g Thiophen in 33,8 ml Chlorbenzol zugetropft. Das Gemisch wurde 2 Stunden bei 0°C nachgerührt, danach 15 Minuten am Rückfluß erhitzt bis die Chlorwasserstoffentwicklung beendet war. Die erhaltene Lösung wurde innerhalb 30 Minuten zu einer auf 70°C erhitzten Suspension von 7,2 g Natriumborhydrid in 50 ml Diglyme getropft. Nach 1,5-stündigem Rühren bei 70°C wurde das Gemisch auf eine Mischung aus 250 g Eis, 200 ml Wasser und 62,5 g konz. wäßr. Salzsäure ausgetragen. Nach Zugabe von 100 ml Chlorbenzol und Durchrühren wurde die organische Phase abgetrennt, mit 100 ml Wasser gewaschen und durch Destillation eingeengt. Ausbeute: 57,7 g Rohsubstanz, 71,0 gew.-%ig (GC) entsprechend einer Ausbeute von 87,2 % d. Th. Durch Destillation wurde beim Siedepunkt 98°C/4 mbar ein 99,4 gew.-%iges 2-(4'-Methylbenzyl)-thiophen erhalten.

### Beispiel 6 2-(4'-Chlorbenzyl)-thiophen

In eine bei 0°C gerührte Suspension von 33,8 g Aluminiumchlorid und 162,5 ml Chlorbenzol wurden 44,2 g 4-Chlorbenzoylchlorid innerhalb 15 Minuten eingetragen. Das Gemisch wurde 90 Minuten nachgerührt. Danach wurde bei 0°C eine Lösung von 21,8 g Thiophen in 33,8 ml Chlorbenzol innerhalb von 30 Minuten unter Rühren zugetropft. Nach 2-stündigem Rühren bei 0°C wurde das Gemisch 15 Minuten am Rückfluß erhitzt, bis die Chlorwasserstoffentwicklung beendet war.

Die erhaltene Lösung wurde anschließend innerhalb 30 Minuten in eine auf 70°C erhitzte Suspension aus 7,2 g Natriumborhydrid und 50 ml Diglyme getropft. Nach 90-minütigem Rühren bei 70°C wurde das Gemisch auf eine Mischung von 250 g Eis, 200 ml Wasser und 62,5 g konz. Salzsäure ausgetragen. Nach Zugabe von 100 ml Chlorbenzol wurde die organische Phase abgetrennt und eingeengt. Ausbeute: 66,2 g Rohsubstanz, 73,4 gew.-%ig (GC). Durch Destillation wurde ein 99,4 gew.-%iges (GC) 2-(4'-Chlorbenzyl)-thiophenyl erhalten, Sdp. 122°C/3,5 mbar.

### Beispiel 7 2-(4'-Fluorbenzyl)-furan

Zu einer Suspension von 33,8 g Aluminiumchlorid in 200 g Fluorbenzol wurde bei Raumtemperatur eine Lösung von 34,4 g Furan-2-carbonsäurechlorid (95 gew.-%ig) in 50 g Fluorbenzol unter Rühren innerhalb 30 Minuten getropft. Das Gemisch wurde auf 60°C erhitzt und 1 Stunde nachgerührt, anschließend noch weitere 90 Minuten bei 80°C. Das so erhaltene Gemisch wurde bei 60°C innerhalb 85 Minuten unter Rühren zu einer Lösung von 22,8 g Dimethylaminboran und 60 g Fluorbenzol getropft. Das Gemisch wurde 80 Minuten bei 70°C nachgerührt, anschließend auf eine Mischung aus 250 g Eis, 200 ml Wasser und 66 g konz. Salzsäure ausgetragen.

Nach kräftigem Durchrühren des Gemisches, Abtrennen und Waschen der organischen Phase mit Wasser sowie Extrahieren der Wasserphase mit Methylenchlorid wurden die vereinigten organischen Phasen eingeengt. Ausbeute: 50,4 g 67 gew.-%iges Produkt (GC), entsprechend 77 % der Theorie. Durch Destillation wurde ein 96 gew.-%iges 2-(4'-Fluorbenzyl)-furan erhalten, Sdp. 70°C/3 mbar.

## Patentansprüche

1. Verfahren zur Herstellung von Methylenverbindungen der Formel
R¹-CH₂R² (I),
in der
R¹ für einen gegebenenfalls durch 1 bis 4 C₁-C₄-Alkylreste, 1 bis 4 Fluor-, Chlor- und/oder Bromatome, eine C₁-C₈-Alkoxygruppe und/oder eine Acetoxygruppe substituierten C₆-C₁₀-Arylrest oder einen gegebenenfalls durch einen C₁-C₄-Alkylrest und/oder 1 Fluor-, Chlor- oder Bromatom substituierten 5 bis 10 C-Atome und ein O- oder S-Atom enthaltenden Heteroarylrest und
R² für einen gegebenenfalls durch 1 bis 5 Fluor-, Chlor- und/oder Bromatome substituierten C₁-C₁₂-Alkylrest, unabhängig von R¹ für einen Rest wie bei R¹ definiert und im Falle R¹ = ein mit 1 bis 4 Fluor-, Chlor- und/oder Bromatomen substituierter C₆-C₁₀-Arylrest auch für einen Furyl- oder Thienylrest stehen,
dadurch gekennzeichnet, daß man einen Aluminiumhalogenid-Komplex der Formel 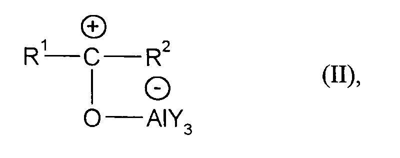 in der
R¹ und R² die oben angegebene Bedeutung haben und
Y für Fluor, Chlor oder Brom steht,
mit einem Aminboran der Formel 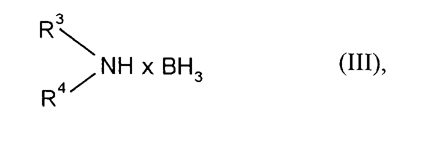 in der
R³ für C₁-C₄-Alkyl und
R⁴ für Wasserstoff oder C₁-C₄-Alkyl stehen
und/oder
mit Natrium- und/oder mit Kaliumborhydrid reduziert.

2. Verfahren nach Anspruch 1, dadurch gekennzeichnet, daß man den Aluminiumhalogenid-Komplex der Formel (II) erhält,
a) durch Friedel-Crafts-Acylierung eines Aromaten oder Heteroaromaten der Formel
R¹-H (IV),
in der
R¹ die in Anspruch 1 angegebene Bedeutung hat,
mit Acylverbindungen der Formel
R²-COX (V),
in der
R² die in Anspruch 1 angegebene Bedeutung hat und
X für Fluor, Chlor oder Brom steht,
oder
b) durch Friedel-Crafts-Acylierung einer Verbindung der Formel
R²-H (VI),
in der
R² die in Anspruch 1 angegebene Bedeutung hat,
mit einer Acylverbindung der Formel
R¹-COX (VII),
in der
R¹ und X die bei Anspruch 1 angegebenen Bedeutungen haben.

3. Verfahren nach Ansprüchen 1 und 2, dadurch gekennzeichnet, daß in den Formeln (I), (II), (V) und (VI) R² für einen Phenylrest steht, der gegebenenfalls 1 bis 3 Substituenten aus der Gruppe Fluor und Chlor enthält oder, wenn R¹ für einen mit 1 bis 4 Fluor-, Chlor- und/oder Bromatomen substituierten C₆-C₁₀-Arylrest steht, auch für einen Furyl- oder Thienylrest steht,
in den Formeln (V) und (VII) X für Chlor oder Brom und unabhängig davon Y in Formel (II) ebenfalls für Chlor oder Brom und
in Formel (III) R³ für Methyl, Ethyl oder tert.-Butyl und
R⁴ für Wasserstoff, Methyl oder Ethyl stehen.

4. Verfahren nach Ansprüchen 1 bis 3, dadurch gekennzeichnet, daß man 2-(4'-Fluorbenzyl)-thiophen, 2-(4'-Chlorbenzyl)-thiophen, 2-(4-Fluorbenzyl)-furan, 2-(2',4'-Dichlor-5-fluorbenzyl)-thiophen, 2-(2'-Chlor-4,5-difluorbenzyl)-thiophen oder 2-(2,4-Dichlor-5-fluorbenzyl)-furan herstellt.

5. Verfahren nach Ansprüchen 1 bis 4, dadurch gekennzeichnet, daß man eine Acylverbindung der Formel (V) bzw. (VII) mit einer Verbindung der Formel (IV) bzw. (VI) unter Zusatz eines Aluminiumhalogenids in einem inerten Lösungsmittel unter Abspaltung von Halogenwasserstoff umsetzt, nach Entfernung des Halogenwasserstoffs das erhaltene Reaktionsgemisch, das einen Aluminiumhalogenid-Komplex der Formel (II) enthält, mit einem Aminboran der Formel (III) oder Natrium- oder Kaliumborhydrid umsetzt, das erhaltene Gemisch zur Eliminierung des Aluminiumhalogenids mit Wasser hydrolysiert, die Wasserphase abtrennt und die organische Phase destilliert.

6. Verfahren nach Ansprüchen 1 bis 5, dadurch gekennzeichnet, daß man die Reduktion von Aluminiumhalogenid-Komplexen der Formel (II) bei 0 bis 100°C durchführt.

7. Verfahren nach Ansprüchen 1 bis 6, dadurch gekennzeichnet, daß man bei der Reduktion von Aluminiumhalogenid-Komplexen der Formel (II) ein zusätzliches Lösungsmittel einsetzt.

8. Verfahren nach Ansprüchen 1 bis 7, dadurch gekennzeichnet, daß man die Reaktion einer Acylverbindung der Formel (V) bzw. (VII) mit einer Verbindung der Formel (IV) bzw. (VI) bei -10 bis +10°C beginnt und im Verlauf der Umsetzung die Temperatur auf 50 bis 110°C anhebt.

9. Verfahren nach Ansprüchen 1 bis 8, dadurch gekennzeichnet, daß man pro Mol Aluminiumhalogenid-Komplex der Formel (II) 0,5 bis 1 Mol der Borwasserstoffverbindung einsetzt.

10. 2-(2',4'-Dichlor-5-fluorbenzyl)-thiophen der Formel
